(19) Europäisches Patentamt
European Patent Office
Office européen des brevets

(11) Veröffentlichungsnummer: **0 073 999**
**B1**

(12) # EUROPÄISCHE PATENTSCHRIFT

(45) Veröffentlichungstag der Patentschrift: **15.01.86**

(51) Int. Cl.⁴: **C 07 D 401/04,** A 01 N 43/00 // C07D249/08

(21) Anmeldenummer: **82107681.7**

(22) Anmeldetag: **23.08.82**

(54) **Hetero-substituierte 4-Pyridon-Derivate, Verfahren zu ihrer Herstellung sowie ihre Verwendung als Herbizide.**

(30) Priorität: **05.09.81 DE 3135186**

(43) Veröffentlichungstag der Anmeldung: **16.03.83 Patentblatt 83/11**

(45) Bekanntmachung des Hinweises auf die Patenterteilung: **15.01.86 Patentblatt 86/3**

(84) Benannte Vertragsstaaten: **AT BE CH DE FR GB IT LI NL**

(56) Entgegenhaltungen:
**DE-A-2 537 753**

**JOURNAL OF ORGANIC CHEMISTRY, Band 44, Nr. 8, 13. April 1979, Seiten 1349-1351, American Chemical Society, US**

(73) Patentinhaber: **BAYER AG, Konzernverwaltung RP Patentabteilung, D-5090 Leverkusen 1 Bayerwerk (DE)**

(72) Erfinder: **Knops, Hans- Joachim, Dr., Claudiusweg 3, D-5600 Wuppertal 1 (DE)**
Erfinder: **Babczinski, Peter, Dr., Bahnstrasse 86, D-5600 Wuppertal 11 (DE)**
Erfinder: **Eue, Ludwig, Dr., Paul- Klee- Strasse 36, D-5090 Leverkusen (DE)**
Erfinder: **Schmidt, Robert, Dr., Im Waldwinkel 110, D-5060 Bergisch- Gladbach 2 (DE)**

**EP 0 073 999 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft neue hetero-substituierte 4-Pyridon-Derivste, ein Verfahren zu ihrer Herstellung sowie ihre Verwendung als Herbizide, insbesondere als selektive Herbizide.

Es ist bereits bekannt geworden, daß 3,5-disubstituierte 4-Pyridone, wie beispielsweise 1-Methyl-3-phenyl-5-(3-trifluormethylphenyl)-4-pyridon, sls Herbizide, wie insbesondere auch zur selektiven Unkrsutbekämpfung in Baumwolle eingesetzt werden können (vergleiche DE-OS 25 37 753). Der selektive Einsatz in verschiedenen anderen Kulturpflanzen ist jedoch nur bedingt möglich, ds es dort zu Schäden kommen kann.

Es wurden neue hetero-substituierte 4-Pyridon-Derivate der allgemeinen Formel I

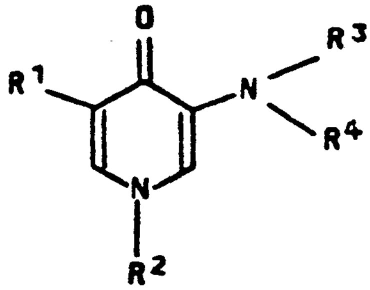

(I)

in welcher

$R^1$ für gegebenenfalls durch Halogen, Alkyl, Alkoxy oder Alkylthio mit jeweils 1 - 4 C-Atomen, oder Halogenalkyl mit 1 - 2 C-Atomen und 1 - 5 gleichen oder verschiedenen Halogenatomen substituiertes Phenyl steht,

$R^2$ für Alkyl mit 1 bis 4 Kohlenstoffatomen, Alkenyl mit 3 bis 4 Kohlenstoffatomen, Alkoxy und Alkoxyalkyl mit jeweils 1 bis 4 Kohlenstoffatomen in jedem Alkylteil, sowie für Dialkylamino mit 1 bis 4 Kohlenstoffatomen in jedem Alkylteil steht und

$R^3$ und $R^4$ gemeinsam mit dem Stickstoffatom, an das sie gebunden sind, für einen gegebenenfalls durch Alkyl oder Alkoxy mit jeweils 1 bis 4 Kohlenstoffatomen oder durch Halogen substituierten 5-gliedrigen heterocyclischen Rest mit 1 bis 3 Stickstoffatomen steht, gefunden. Weiterhin wurde gefunden, daß man die hetero-substituierten 4-Pyridon-Derivate der Formel (1) erhält, wenn man Ketone der Formel II

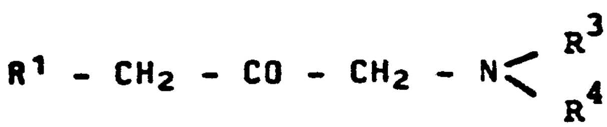

(II)

in welcher

$R^1$, $R^3$ und $R^4$ die oben angegebene Bedeutung

haben,

zunächst mit einem Formylierungsmittel in Gegenwart einer Base und in Gegenwart eines Verdünnungsmittels umsetzt, danach mit Salzen von Aminen der Formel III

$R^2 - NH_2$ III

in welcher

$R^2$ die oben angegebene Bedeutung hat,

in Gegenwart desselben Verdünnungsmittels in üblicher Weise versetzt und anschließend das Gemisch der beiden entstehenden Enamine der Formeln IVa und IVb

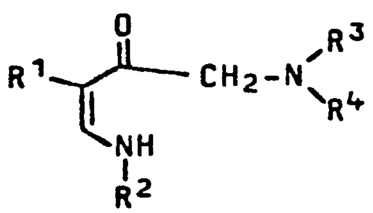

und

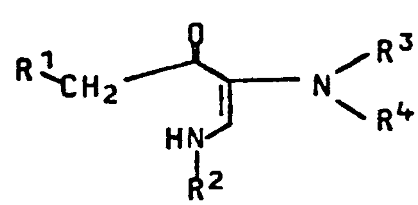

(IVa) (IVb)

in welchen
$R^1$, $R^2$, $R^3$ und $R^4$ die oben angegebene Bedeutung haben,
ohne Isolierung der gleichen Reaktionsfolge - Umsetzung mit dem gleichen Formylierungsmittel und dem gleichen Aminsalz unter den gleichen Reaktionsbedingungen - unterwirft.

Außerdem wurde gefunden, daß die hetero-substituierten 4-Pyridon-Derivate der Formel (I) gute herbizide, insbesondere selektiv-herbizide Eigenschaften aufweisen.

Die erfindungsgemäßen Wirkstoffe stellen somit eine Bereicherung der herbiziden Mittel, insbesondere der selektiven chemischen Unkrautbekämpfung dar.

Die erfindungsgemäßen hetero-substituierten 4-Pyridon-Derivate sind durch die Formel (I) allgemein definiert. In dieser Formel stehen vorzugsweise:

$R^1$ für gegebenenfslls einfach oder zweifach, gleich oder verschieden substituiertes Phenyl, wobei als Substituenten gensnnt seien: Fluor, Chlor, Brom, Methyl, Ethyl, Isopropyl, Methoxy, Ethoxy, Isopropoxy, Methylthio, Ethylthio, Isopropylthio und Trifluormethyl;

$R^2$ für Methyl, Ethyl, i-Propyl, Allyl, Propargyl, Methoxy, Ethoxy, Methoxymethyl, Ethoxymethyl, Ethoxyethyl und Dimethylamino und

$R^3$ und $R^4$ gemeinsam mit dem Stickstoffatom, an das sie gebunden sind, für folgende, gegebenenfalls durch Methyl, Methoxy oder Chlor substituierte heterocyclischen Reste: 1,2,4-Triazolyl, Imidazolyl und Pyrazolyl.

Verwendet man beispielweise 1-(3-Trifluormethylphenyl)-3-(1,2,4-triazol-1-yl)-propanon als Ausgangsstoff, Ameisensäureethylester als Formylierungsmittel und Methylamin-hydrochlorid als Aminkomponente, so kann der Reaktionsablauf durch das folgende Formelschema wiedergegeben werden:

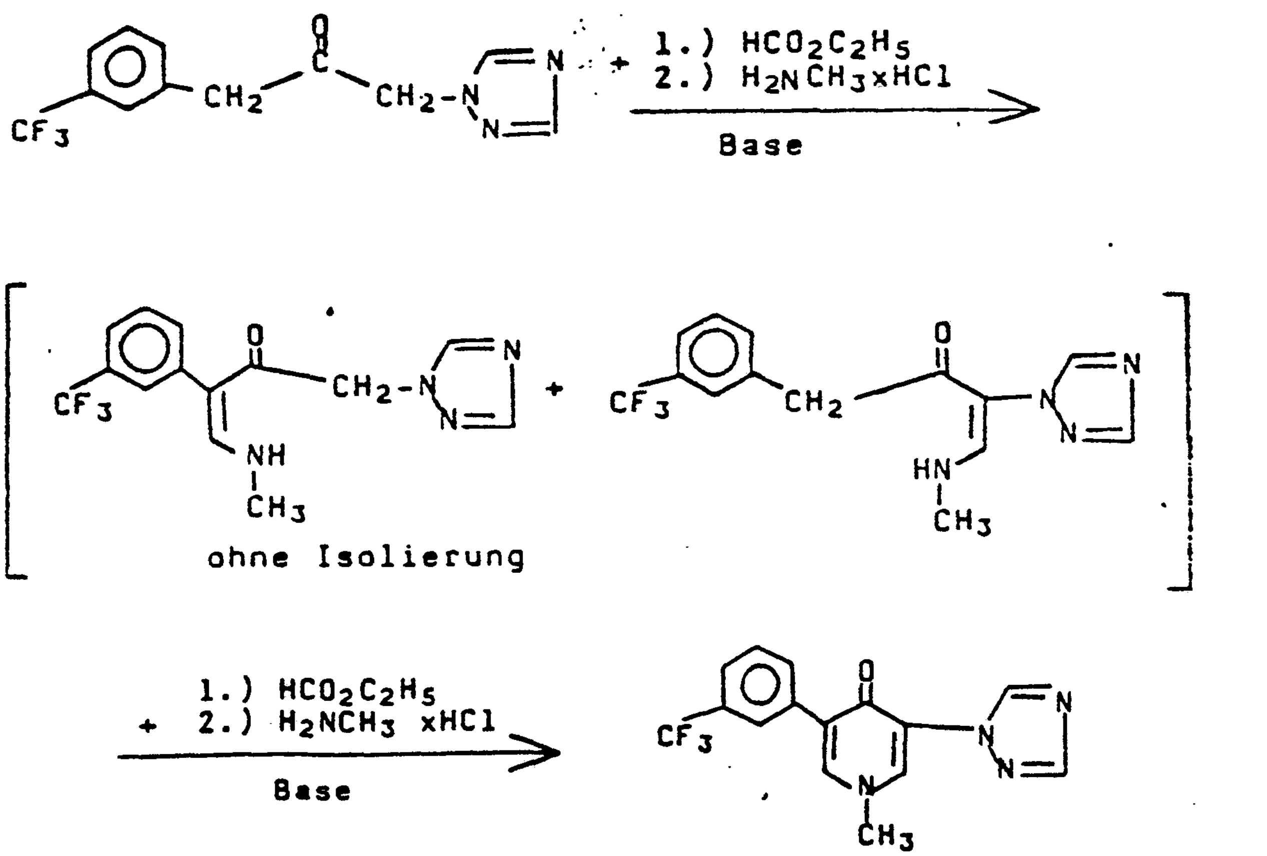


Die für die erfindungsgemäße Reaktion als Ausgangsstoffe zu verwendenden Ketone sind durch die Formel (II) allgemein definiert. In dieser Formel stehen $R^1$, $R^3$ und $R^4$ vorzugsweise für die Reste, die bereits im Zusammenhang mit der Beschreibung der erfindungsgemäßen Stoffe der Formel (I) vorzugsweise für diese Substituenten genannt wurden.

Die Ketone der Formel (II) sind bekannt, bzw. können sie in bekannter Art und Weise erhalten werden, indem man z.B. Benzylcyanide der Formel IV

$R^1 - CH_2 - CN$ (IV)

in welcher

$R^1$ die oben angegebene Bedeutung hat,

mit Essigsäureester-Derivaten der Formel V

$RO - CO - CH_2NR^3R^4$ (V)

in welcher
R für Alkyl mit 1 bis 4 Kohlenstoffatomen steht und
$R^3$ und $R^4$ die oben angegebene Bedeutung haben,
in Gegenwart einer Base, wie z.B. Natriumethylat, und in Gegenwart eines inerten organischen Lösungsmittels, wie z.B. Ethanol, vorzugsweise bei der Siedetemperatur des verwendeten Lösungsmittels, umsetzt und anschließend im System Schwefelsäure/Wasser hydrolisiert (vergleiche auch die Herstellungsbeispiele).

Die Benzylcyanide der Formel (IV) und die Essigsäureester-Derivate der Formel (V) sind allgemein bekannte Verbindungen der organischen Chemie bzw. können sie in üblicher Art und Weise hergestellt werden

Als Formylierungsmittel können für die erfindungsgemäße Reaktion alle üblicherweise verwendbaren Formylierungsreagenzien eingesetzt werden. Hierzu gehören vorzugsweiae Ameisensäureester, wie z.B. Ameisensäuremethyl- und -ethylester.

Die außerdem für die erfindungsgemäße Reaktion als Ausgangsstoffe zu verwendenden Amine sind durch die Formel (III) allgemein definiert. In dieser Formel steht $R^2$ vorzugsweise für die Reste, die bereits im Zusammenhang mit der Beschreibung der erfindungsgemäßen Stoffe der Formel (I) vorzugsweise für diesen Substituenten genannt wurden. Die Amine der Formel (III) werden bevorzugt in Form ihrer Salze eingesetzt, wie z.B. als Hydrohalogenide oder Hydrosulfate.

Die Amine der Formel (III) sind allgemein bekannte Verbindungen der organischen Chemie.

Die erfindungsgemäße Reaktion wird in Gegenwart einer Base durchgeführt. Hierbei können alle üblicherweise verwendbaren organischen und anorganischen Basen eingesetzt werden, wie insbesondere Alkoholate, wie z.B. Natriummethylat oder -ethylat und Kalium-tert.-butylat, oder Natrium- bzw. Kaliumhydrid.

Als Verdünnungsmittel kommen für die erfindungsgemäße Umsetzung vorzugsweise mit Wasser mischbare organische Lösungsmittel infrage. Hierzu gehören vorzugsweise Ether, wie insbesondere Tetrahydrofuran oder Dioxan. Die Reaktionstemperaturen können bei der Durchführung des erfindungsgemäßen Verfahrens in einem bestimmten Bereich variiert werden. Im allgemeinen arbeitet man zwischen -20 und +60°C, vorzugsweise bei -10 bis 40°C.

Bei der Durchführung des erfindungsgemäßen Verfahrens setzt man zunächst auf 1 Mol Keton der Formel (II) einen Ueberschuß an Formylierungsmittel, insbesondere die zweibis dreifache Menge, einen Ueberschuß an Base, insbesondere die zwei- bis dreifache Menge, und einen Ueberschuß an Amin der Formel (III), insbesondere ebenfalls die zwei- bis dreifache Menge, ein. Die gleichen überschüssigen Mengen an Formylierungsmittel, Base und Amin werden bei der Wiederholung der erfindungsmaßigen Reaktionsfolge (Ringschlußreaktion) eingesetzt. Die Aufarbeitung und Isolierung der Endprodukte der Formel (I) erfolgt in allgemein üblicher Art und Weise.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vitia, Nicotiana, Lycopersicon, Arachis, Brassica, Lactuca, Cucumis. Cucurbita.

Monokotyle Unkräuter der Gattungen: Echinochloa, Setaria, Paricum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Ele charis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hord um, Avena, Secale, Sorghum, Panicum, Saccharum, Ananas, Asparagus, Allium.

Die Verwendung der erfindungsgemäßen Wirkstoffe ist jedoch eineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrieund Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Wein-, Citrus-, Nuss-, Bananen-, Kaffee-, Tee-, Gummi-, Oelpalm-, Kakao-, Beerenfrucht- und Hopfenanlagen und zur selektiven Unkrautbekämpfung in einjährigen Kulturen eingesetzt werden.

Die erfindungsgemäßen Wirkstoffe zeigen neben einer sehr guten allgemeinen herbizide Wirkung eine gute Verträglichkeit gegenüber Nutzpflanzen. So ist es möglich, wichtige Schadgräser selektiv in wichtigen Kulturpflanzen, wie z.B. in Baumwolle, Weizen, Sojabohnen und Mais zu bekämpfen.

Die Wirkstoffe können in die üblichen Formulierungen übergeführt werden, wie Lösungen, Emulsionen, Spritzpulver Suspensionen, Pulver, Stäubemittel, Pasten, lösliche Pulver, Granulate, Suspensions-Emulsions-Konzentrate, wirkstoffimprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung

von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silicate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %. Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen,Spritzen, Sprühen, Streuen oder Stäuben.

Die erfindungsgemäßen Wirkstoffe können sowohl vor als auch nach dem Auflaufen der Pflanzen appliziert werden. Die Anwendung wird vorzugsweise vor dem Auflaufen der Pflanzen, also im pre-emergence-Verfahren, vorgenommen. Sie können auch vor der Saat in den Boden eingearbeitet werden.

Die aufgewandte Wirkstoffmenge kann in größeren Bereichen schwanken. Sie hängt im wesentlichen von der Art des gewünschten Effektes ab. Im allgemeinen liegen die Aufwandmengen zwischen 0,1 und 10 kg Wirkstoff pro ha, vorzugsweise zwischen 0,1 und 5 kg/ha.

Die nachfolgenden Beispiele dienen zur Erläuterung der Erfindung.

**Herstellungsbeispiele**

**Beispiel 1**

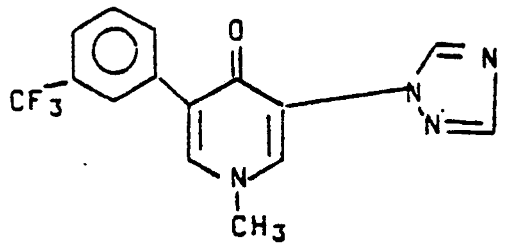

(1)

**Zu 7,8 g 80 %-igem Natriumhydrid in 100 ml absolutem Tetrahydrofuran werden bei -5°C bis 0°C 27g 1-(3-Trifluormethylphenyl)-3-(1,2,4-triazol-1-yl)-propanon in 100 ml Tetrahydrofuran getropft. Zu dieser Reaktionslösung werden bei gleicher Temperatur 20,3 ml Ameisensäureethylester gegeben. Nach einer weiteren Stunde wird nochmals mit 16,5 ml Ameisensäureethylester versetzt. Man läßt auf Raumtemperatur erwärmen und über Nacht rühren. Danach werden 16,8 g Methylamin-hydrochlorid in 50 ml Wasser zugegeben, wobei die**

Temperatur auf 30°C steigt. Man hält das Reaktionsgemisch eine halbe Stunde bei dieser Temperatur. Nach dem Abkühlen wird die filtrierte Lösung mit 200 ml Methylenchlorid und 100 ml Wasser versetzt, die organische Phase abgetrennt, nochmals mit Wasser gewaschen, über Natriumsulfat getrocknet und eingeengt. Der Rückstand, ein Gemisch der beiden Enamine, wird erneut der gleichen Reaktion unterworfen. Der dann erhaltene Rückstand wird mit Ether versetzt und der kristalline Niederschlag abgesaugt. Man erhält 8 g (25 % der Theorie) 1-Methyl-3-(1,2,4-triazol-1-yl)-5-(3-trifluormethylphenyl)-4-pyridon vom Schmelzpunkt 181-83°C.

**Herstellung des Ausgangsproduktes**

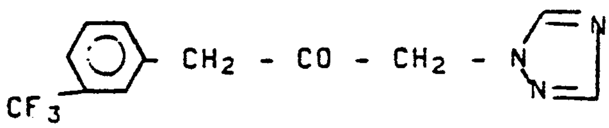

266,3g 1-Cyano-1-(3-trifluormethylphenyl)-3-(1 2 4-tri-azol-1-yl)-propanon-hydrochlorid werden mit 320 ml konzentrierter Schwefelsäure und 94 ml Wasser versetzt und 1,5 Stunden auf 100 °C erhitzt. Nach Beendigung der starken $CO_2$-Entwicklung läßt man abkühlen und versetzt mit 940 ml Wasser. Nach kurzer Zeit bei 0 bis 20°C kristallisiert das Sulfat des gewünschten Endproduktes aus. Dieses wird in Wasser aufgeschlämmt und mit Natriumhydrogencarbonatlösung auf einen pH-Wert von 7-8 eingestellt. Der entstehende kristalline Niederschlag wird abgesaugt, in Chloroform gelöst, über Natriumsulfat getrocknet und eingeengt. Man erhält 131,3g (72 % der Theorie) 1-(3-Trifluormethylphenyl)-3-(1,2,4-triazol-1-yl)-propanon vom Schmelzpunkt 85-87°C.

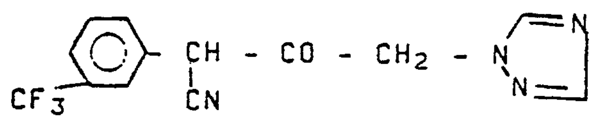

128g 1,2,4-Triazolylessigsäureethylester und 123,3g 3-Tri-fluormethylbenzylcyanid werden innerhalb von 6 Stunden in eine siedende Lösung von 30,6g Natrium in 500ml Ethanol getropft und anschließend 12 Stunden unter Rückfluß erhitzt. Der nach dem Abkühlen ausgefallene Niederschlag wird in Wasser gelöst und zur Entfernung nicht umgesetzter Edukte mit Ether extrahiert. Die wässrige Phase wird mit verdünnter Salzsäure bis pH 3 angesäuert, der Niederschlag abgesaugt, das Filtrat mit Ether extrahiert,der Extrakt getrocknet und eingeengt. Die Niederschlag werden vereinigt. Man erhält 266g 1-Cyano-1-(3-trifluor-methylphenyl)-3-(1,2,4-triazol-1-yl)-propanon-hydrochlorid vom Schmelzpunkt 95-98°C.

In entsprechender Weise werden auch die in der folgenden Tabelle 1 aufgeführten Verbindungen der allgemeinen Formel

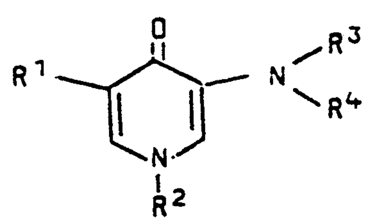

(I)

erhalten:

**Tabelle 1**

| Beispiel Nr. | $R^1$ | $R^2$ | $-NR^3R^4$ | Schmelzpunkt (°C) |
|---|---|---|---|---|
| 2 | 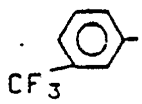 | $-OCH_3$ | 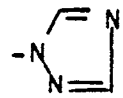 | 262-70 |
| 3 | 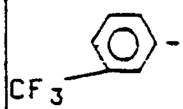 | $-N(CH_3)_2$ | 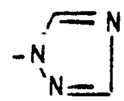 | 147-49 |
| 4 | 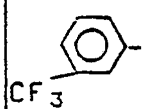 | $-CH_2CH=CH_2$ | 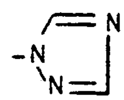 | 96-102 |
| 5 | 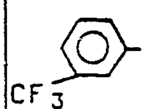 | $-CH_2CH_2OCH_3$ | 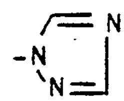 | Oel |
| 6 | 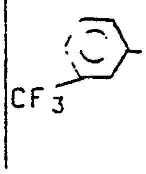 | $-C_2H_5$ | 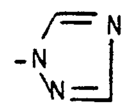 | 171-72 |

Tabelle 1 (Fortsetzung)

| Beispiel Nr. | $R^1$ | $R^2$ | $-NR^3R^4$ | Schmelzpunkt (°C) |
|---|---|---|---|---|
| 7 | 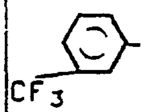 | $-C_3H_7-n$ | 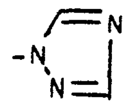 | 109 - 10 |
| 8 | 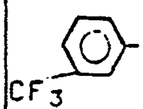 | $-C_3H_7-i$ | 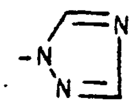 | 134 - 35 |
| 9 | 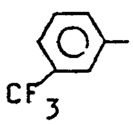 | $-CH_3$ | 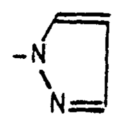 | 97 |
| 10 | 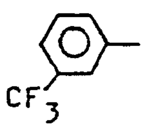 | $-CH_3$ | 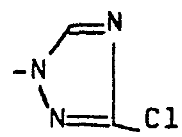 | 164 - 165 |
| 11 | 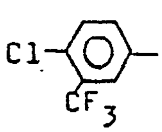 | $-CH_3$ | 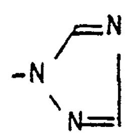 | 189 - 190 |
| 12 | 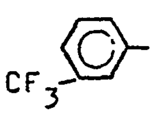 | $-CH_3$ | 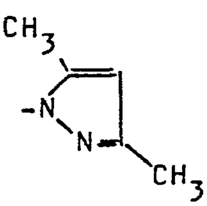 | |

**Verwendungsbeispiele:**

**Beispiel A**

Pre-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die

Aufwandmenge des Wirkstoffes pro Flächeneinheit. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)

100 % = totale Vernichtung

In diesem Test zeigt z.B. die Verbindung gemäß Herstellungsbeispiel 1 neben einer sehr guten allgemeinen herbiziden Wirkung eine gute Verträglichkeit in Nutzpflanzen, wie Mais, Sojabohnen, Weizen und Baumwolle.

**Patentansprüche:**

1. Hetero-substituiertes 4-pyridon-Derivate der allgemeinen Formel I

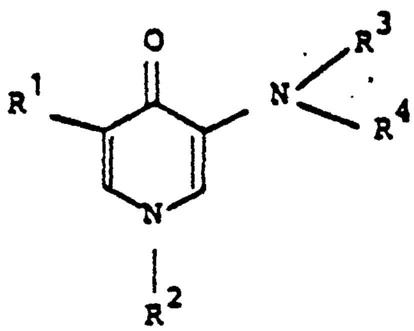

(I)

dadurch gekennzeichnet, daß

$R^1$ für gegebenenfalls durch Halogen, Alkyl, Alkoxy oder Alkylthio mit jeweils 1 - 4 C-Atomen, oder Halogenalkyl mit 1 - 2 C-Atomen und 1 - 5 gleichen oder verschiedenen Halogenatomen substituiertes Phenyl steht,

$R^2$ für Alkyl mit 1 bis 4 Kohlenstoffatomen, Alkenyl mit 3 bis 4 Kohlenstoffatomen, Alkoxy und Alkoxyalkyl mit jeweils 1 bis 4 Kohlenstoffatomen in jedem Alkylteil, sowie für Dialkylamino mit 1 bis 4 Kohlenstoffatomen in jeden Alkylteil steht und

$R^3$ und $R^4$ gemeinsam mit dem Stickstoffatom, an das sie gebunden sind, für einen gegebenenfalls durch Alkyl oder Alkoxy mit jeweils 1 bis 4 Kohlenstoffatomen oder durch Halogen substituierten 5-gliedrigen heterocyclischen Rest mit 1 bis 3 Stickstoffatomen stehen.

2. 1-Methyl-3-(1,2,4-triazol-1-yl)-5-(3-trifluormethyl-phenyl)-4-pyridon der Formel

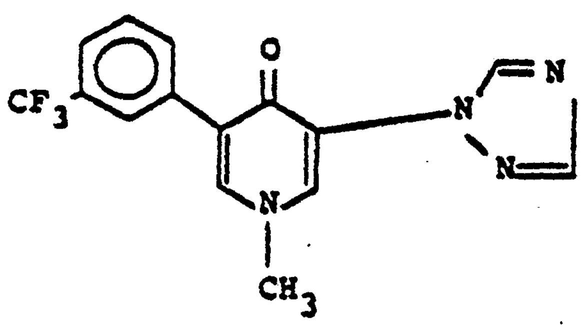

(1)

gemäß Anspruch 1.

3. Verfahren zur Herstellung von hetero-substituierten 4-pyridon-Derivaten der allgemeinen Formel (I)

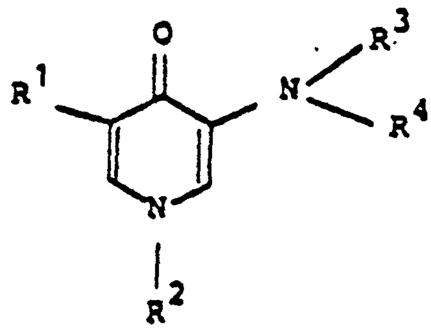

(I)

in welcher

$R^1$ für gegebenenfalls durch Halogen, Alkyl, Alkoxy oder Alkylthio mit jeweils 1 - 4 C-Atomen, oder Halogenalkyl mit 1 - 2 C-Atomen und 1 - 5 gleichen oder verschiedenen Halogenatomen substituiertes Phenyl steht,

$R^2$ für Alkyl mit 1 bis 4 Kohlenstoffatomen, Alkenyl mit 3 bis 4 Kohlenstoffatomen, Alkoxy und Alkoxyalkyl mit jeweils 1 bis 4 Kohlenstoffatomen in jedem Alkylteil, sowie für Dialkylamino mit 1 bis 4 Kohlenstoffatomen in jedem Alkylteil steht und

$R^3$ und $R^4$ gemeinsam mit dem Stickstoffatom, an das sie gebunden sind, für einen gegebenenfalls durch Alkyl oder Alkoxy mit jeweils 1 bis 1 Kohlenstoffatomen oder durch Halogen substituierten 5-gliedrigen heterocyclischen Rest mit 1 bis 3 Stickstoffatomen stehen,

dadurch gekennzeichnet, dass man Ketone der Formel II

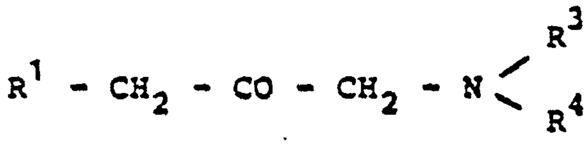

(II)

in welcher

$R^1$,$R^2$ und $R^3$ die oben angegebene Bedeutung haben,

zunächst mit einem Formylierungsmittel in Gegenwart einer Base und in Gegewart eines Verdünnungsmittels umsetzt, danach mit Salzen von Aminen der Formel III

$R^2 - NH_2$ (III)

in welcher

$R^2$ die oben angegebene Bedeutung hat,

in Gegenwart desselben Verdünnungsmittels in üblicher Weise versetzt und anschließend das Gemisch der bei den entstehenden Enamine der Formeln IVa und IVb

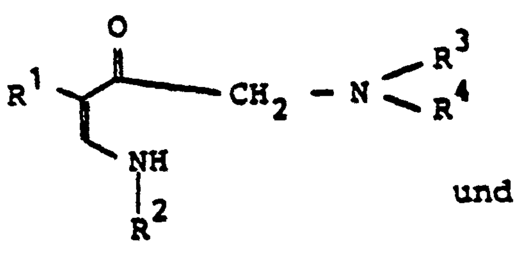

und

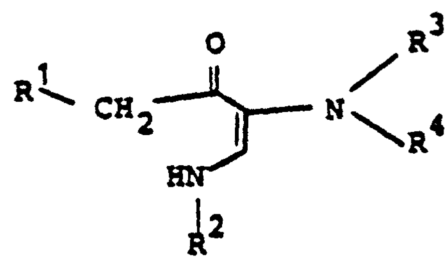

(IVa) (IVb)

in welchen

$R^1$, $R^2$, $R^3$ und $R^4$ die oben angegebene Bedeutung haben,

ohne Isolierung der gleichen Reaktionsfolge -Umsetzung mit dem gleichen Formylierungsmittel und dem gleichen Aminsalz unter den gleichen Reaktionsbedingungen unterwirft.

4. Herbizide Mittel, gekennzeichnet durch einen Gehalt an mindestens einem hetero-substituierten 4-Pyridon-Derivat der Formel (I) gemäß Anspruch 1 und 3.

5. Verfahren zur Bekämpfung von unerwünschtem Pflanzenwachstum, dadurch gekennzeichnet, daß man heterosubstituierte 4-pyridon-Derivate der Formel (I) gemäß Anspruch 1 und 3 auf die unerwünschten Pflanzen oder ihren Lebensraum einwirken läßt.

6. Verwendung von hetero-substituierten 4-Pyridon-Deriva-ten der Formel (I) gemäß Anspruch 1 und 3 zur Bekämpfung von unerwünschtem Pflanzenwachstum.

7. Verfahren zur Herstellung Von herbiziden Mitteln, dadurch gekennzeichnet, daß man hetero-substituiete 4-Pyridon-Derivate der Formel (I) gemäß Anspruch 1 und 3 mit Streckmitteln und/oder oberflächenaktiven Mitteln Vermischt.

**Claims**

1. Hetero-substituted pyrid-4-one derivatives of the general formula I

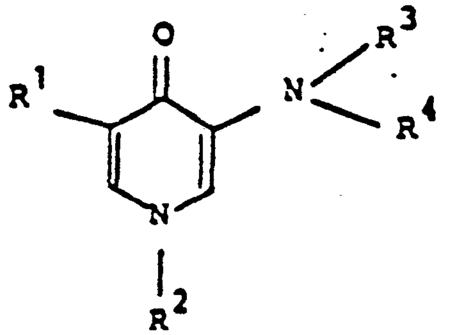

(I)

characterised in that

$R^1$ represents phenyl which is optionally substituted by halogen, alkyl, alkoxy or alkylthio with in each case 1 - 4 C atoms, or halogenoalkyl with 1 - 2 C atoms and 1 - 5 identical or different halogen atoms,

$R^2$ represents alkyl with 1 to 4 carbon atoms, alkenyl with 3 to 4 carbon atoms, alkoxy and alkoxyalkyl with in each case 1 to 4 carbon atoms in each alkyl part, and dialkylamino with 1 to 4 carbon atoms in each alkyl part and

$R^3$ and $R^4$, together with the nitrogen atom to which they are bonded, represent a 5-membered heterocyclic radical which has 1 to 3 nitrogen atoms and is optionally substituted by alkyl or alkoxy with in each case 1 to 4 carbon atoms or by halogen.

2. 1-Methyl-3-(1,2,4-triazol-1-yl)-5-(3-trifluoromethylphenyl)-pyrid-4-one of the formula

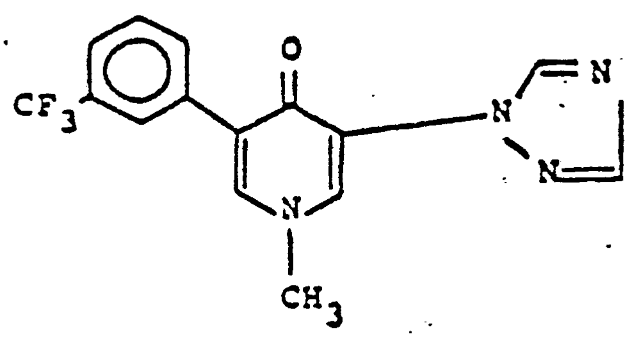

(1)

according to Claim 1.

3. Process for the preparation of hetero-substituted pyrid-4-one derivatives of the general formula (I)

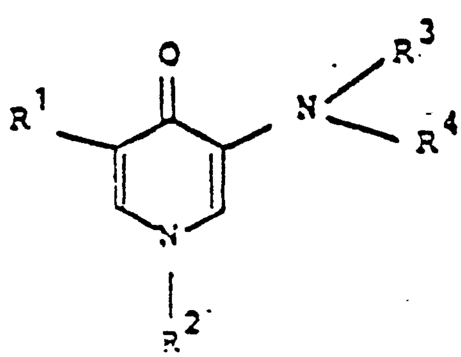

(I)

in which

$R^1$ represents phenyl which is optionally substituted by halogen, alkyl, alkoxy or alkylthio with in each case 1 - 4 C atoms, or halogenoalkyl with 1 - 2 C atoms and 1 - 5 identical or different halogen atoms,

$R^2$ represents alkyl with 1 to 4 carbon atoms, alkenyl with 3 to 4 carbon atoms, alkoxy and alkoxyalkyl with in each case 1 to 4 carbon atoms in each alkyl part, and dialkylamino with 1 to 4 carbonatoms in each alkyl part and

$R^3$ and $R^4$, together with the nitrogen atom to which they are bonded, represent a 5-membered heterocyclic radical which has 1 to 3 nitrogen atoms and is optionally substituted by alkyl or alkoxy with in each case 1 to 4 carbon atoms or by halogen,

characterised in that ketones of the formula II

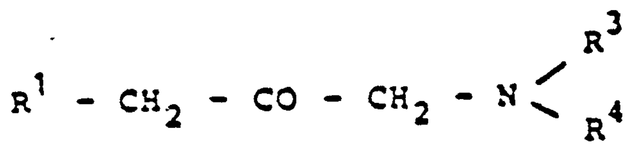

(II)

in which

$R^1$, $R^3$ and $R^4$ have the meaning given above, are first reacted with a formylating agent in the presence of a base and in the presence of a diluent, and then salts of amines of the formula III

$R^2 - NH_2$ (III)

in which

$R^2$ has the meaning given above,

are added in the customary manner in the presence of the same diluent, and then the mixture of the two resulting enamines of the formulae IVa and IVb

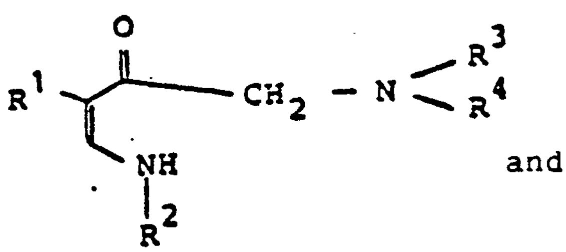

(IVa)

and

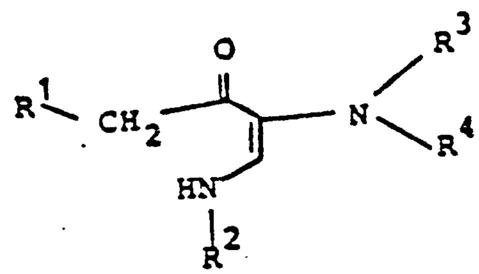

(IVb)

in which

$R^1$, $R^2$, $R^3$ and $R^4$ have the meaning given above, is subjected, without isolation, to the same reaction sequencereaction with the same formylating agent and the same amine salt under the same reaction conditions.

4. Herbicidal agents, characterised in that they contain at least one hetero-substituted pyrid-4-one derivative of the formula (I) according to Claim 1 and 3.

5. Process for combating undesired plant growth, characterised in that hetero-substituted pyrid-4-one derivatives of the formula (I) according to Claim 1 and 3 are allowed to act on the undesired plants or their environment.

6. Use of hetero-substituted pyrid-4-one derivatives of the formula (I) according to Claim 1 and 3 for combating undesired plant growth.

7. Process for the preparation of herbicidal agents, characterised in that hetero-substituted pyrid-4-one derivatives of the formula (1) according to Claim 1 and 3 are mixed with extenders and/or surface-active agents.

**Revendications**

1. Dérivés de 4-pyridone hétéro-substitués de formule générale I:

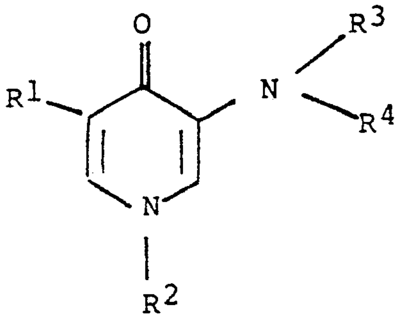

(I)

caractérisés en ce que

$R^1$ représente un phényle éventuellement substitué par de l'halogène, alcoyle, alcoxy ou alcoylthio ayant chacun 1 à 4 atomes de carbone, ou halogénoalcoyle ayant 1 à 2 atomes de carbone et 1 à 5 atomes d'halogène, identiques ou différents,

$R^2$ représente un alcoyle ayant 1 à 4 atomes de carbone, alcényle ayant 3 à 4 atomes de carbone, alcoxy et alcoxyalcoyle ayant chacun 1 à 4 atomes de carbone dans chaque portion alcoyle, de même que dialcoylamino ayant 1 à 4 atomes de carbone dans chaque portion alcoyle et

$R^3$ et $R^4$, en commun avec l'atome d'azote auquel ils sont attachés, représentent un radical hétérocyclique à 5 chainons comportant 1 à 3 atomes d'azote et éventuellement substitué par un alcoyle ou alcoxy ayant chacun 1 à 4 atomes de carbone ou par de l'halogène.

2. 1-méthyl-3-(1,2,4-triazol-1-yl)-5-(3-trifluorométhylphényl)-4-pyridone

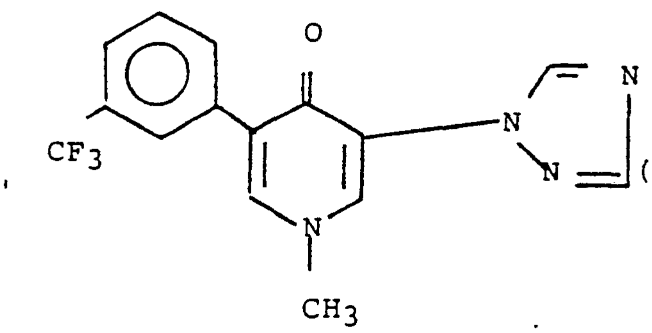

(1)

selon la revendication 1.

3. Procédé de fabrication de dérivés de 4-pyridone hétéro-substitués de formule générale (I)

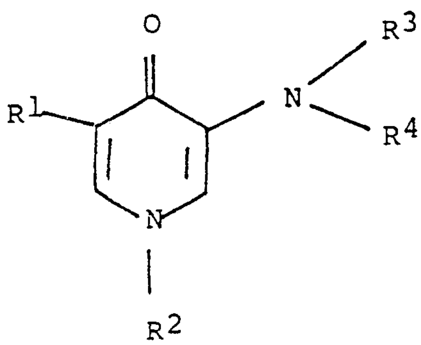

(I)

dans laquelle

$R^1$ représente un phényle éventuellement subsr·tué par de l'halogène, alcoyle, alcoxy ou alcoylthio ayant chacun 1 à 4 atomes de carbone, ou halogénoalcoyle ayant 1 è 2 atomes de carbone et 1 à 5 atomes d'halogene, identiques ou différents,

$R^2$ représente un alcoyle ayant 1 à 4 atomes de carbone, alcényle ayant 3 à 4 atomes de carbone, alcoxy et alcoxyalcoyle ayant chacun 1 à 4 atomes de carbone dans chaque portion alcoyle, de même que dialcoylamino ayant 1 à 4 atomes de carbone dans chaque portion alcoyle,

et $R^3$ et $R^4$ en commun avec l'atome d'azote auquel ils sont attachés, représentent un radical hétérocyclique à 5 chaînons qui comporte 1 à 3 atomes d'azote et qui est éventuellement substitué par un alcoyle ou alcoxy ayant chacun 1 à 4 atomes de carbone ou par de l'halogène,

caractérisé en ce qu'on fait réagir des cétones de formule II

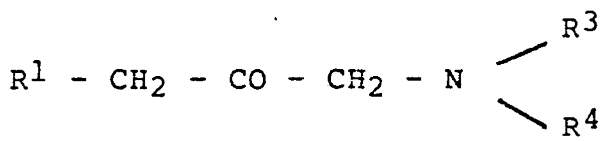

(II)

dans laquelle

$R^1$, $R^3$ et $R^4$ ont la signification indiquée plus haut, tout d'abord avec un agent de formylation en présence d'une base et en présence d'un diluant, on ajoute après cela des sels d'amines de formule III

$R^2 - NH_2$ (III)

dans laquelle

$R^2$ a la signification indiquée plus haut,

en présence du même diluant et de la manière usuelle, puis on soumet le mélange des deux ènamines obtenues de formules IVa et IVb

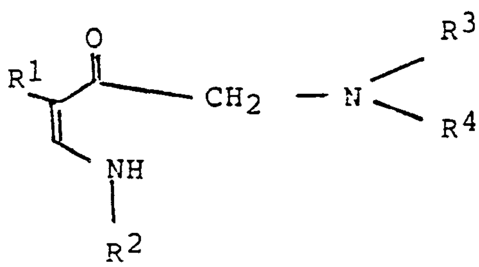

et

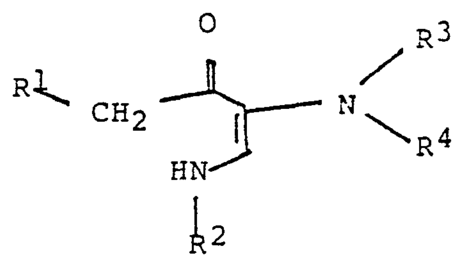

(IVa) (IVb)

dans lesquelles

$R^1$, $R^2$, $R^3$ et $R^4$ ont la signification indiquée plus haut, sans isolation, à la même série de réactions: réaction avec le même agent de formylation et le même sel d'amine, dans les mêmes conditions de réaction.

4. Agent herbicide, caractérisé par une teneur en au moins un dérivé de 4-pyridone hétérosubstitué de formule (I) selon les revendications 1 et 3.

5. Procédé pour combattre la croissance indésirable des plantes, caractérisé en ce qu'on fait agir des dérivés de 4-pyridone hétéro-substitués de formule (I) selon les revendications 1 et 3 sur les plantes non désirées ou sur leur espace vital.

6. Utilisation de dérivés de 4-pyridone hetéro-substitues de formule (I) selon les revendications 1 et 3 pour combattre la croissance indesirable des plantes.

7. Procédé de fabrication d'agents herbicides, caractérisé en ce qu'on mélange des dérivés de 4-pyridone hétéro-substitués de formule (I) selon les revendications 1 et 3 avec des diluants et/ou des agents tensioactifs.